# EUROPÄISCHE PATENTSCHRIFT

(11) **EP 1 264 828 B1**
(45) Veröffentlichungstag und Bekanntmachung des Hinweises auf die Patenterteilung: **14.04.2004**
(21) Anmeldenummer: 02016038.8
(22) Anmeldetag: 20.07.1999
(51) Int. Cl.: A61K 31/495, A61P 35/00

(54) **Urokinase-Inhibitoren**
Inhibitor of urokinase
Inhibiteur d'urokinase

(30) Priorität: 20.07.1998 EP 98113519
(43) Veröffentlichungstag der Anmeldung: 11.12.2002
(62) Teilanmeldung aus: 99936570.3
(73) Patentinhaber: Wilex AG, 81675 München (DE)
(72) Erfinder: Wilhelm, Olaf, 81545 München (DE); Magdolen, Viktor, 85551 Kirchheim (DE); Stürzebecher, Jörg, 99094 Erfurt-Rhoda (DE); Foekens, John, 2908-XA Capelle a/d Yssel (NL); Lutz, Verena, 81475 München (DE)
(74) Vertreter: Weiss, Wolfgang, Dipl.-Chem. Dr.

(56) Entgegenhaltungen:
- WO-A-00/17158
- WO-A-96/05189
- CH-A- 689 611
- STURZEBECHER J ET AL: "3-Amidinophenylalanine-based Inhibitors of Urokinase" BIOORGANIC & MEDICINAL CHEMISTRY LETTERS, OXFORD, GB, Bd. 9, Nr. 21, 1. November 1999 (1999-11-01), Seiten 3147-3152, XP004181024 ISSN: 0960-894X

## Beschreibung

Die Erfindung betrifft die Verwendung von Nα-(2,4,6-Triisopropyl-phenylsulfonyl)-3-amidino-(D,L)-phenylalanin-4-ethoxycarbonyl-piperazid-hydrochlorid bzw. des L-Entantiomers davon oder eines pharmazeutisch verträglichen Salzes dieser Verbindungen als Urokinase-Inhibitoren insbesondere zur Behandlung von malignen Tumoren und der Metastasierung bzw. als Mittel zum Targeting von Lymphzellen und zur Behandlung von Erkrankungen des Lymphgewebes, insbesondere von Lymphomen.

Die Fähigkeit solider Tumoren zur Ausbreitung und Metastasierung in umgebendes Gewebe korreliert mit dem Abbau bzw. Umbau der extrazellulären Matrix (Tumorstroma) in der Umgebung der Tumorzelle, bzw. mit deren Fähigkeit zur Durchdringung der Basalmembran. Obwohl die (patho)biochemischen Zusammenhänge noch nicht endgültig aufgeklärt sind, kommen dem Plasminogenaktivator Urokinase (uPA) und dem Urokinaserezeptor (uPAR) eine zentrale Bedeutung zu. uPA vermittelt die proteolytische Spaltung von Plasminogen zu Plasmin. Plasmin wiederum ist eine Protease mit breitem Wirkspektrum, die Komponenten der extrazellulären Matrix, wie Fibrin, Fibronektin, Laminin und das Proteingerüst der Proteoglykane, direkt abzubauen vermag. Außerdem kann Plasmin "latente" Metalloproteasen und das inaktive Proenzym von uPA, pro-uPA, aktivieren.

Tumorzellen und nichtmaligne Zellen des Tumorstromas synthetisieren und sezernieren das enzymatisch inaktive Proenzym pro-uPA. Proteasen, wie z.B. Plasmin oder die Kathepsine B und L, spalten pro-uPA durch limitierte Proteolyse zur aktiven Serinprotease HMW-uPA (HMW=high molecular weight). pro-uPA und die aktive Protease HMW-uPA binden an den Zelloberflächenrezeptor uPAR (CD87). Plasmin(ogen) bindet ebenfalls an spezifische Rezeptoren auf der Plasmamembran der Tumorzelle, wodurch eine Fokussierung und Amplifikation der Plasminogenaktivierung in der direkten Umgebung der Tumorzelle erreicht wird. Invasiven Zellen ist somit die Möglichkeit gegeben, die extrazelluläre Matrix abzubauen, ohne sich der für eine gerichtete Bewegung notwendigen Unterlagen durch Proteolyse zu entziehen.

In verschiedenen zellbiologischen Studien konnte gezeigt werden, dass dem zellassoziierten Plasminogenaktivator-Systeminnerhalb der kaskadenartigen Reaktionswege tumorassoziierter Proteolysesyteme ein besonderer Stellenwert zukommt (Wilhelm et al. (1994) The Urokinase/Urokinase receptor system: A new target for cancer therapy? In: Schmitt M., Graeff H., Kindermann G. (Hrsg): Prospects in Diagnosis and Treatment of Cancer. International Congress Series, Excerpta Medica 1050, Amsterdam, Elsevier 1994, pp145-156). An Kulturen humaner Kolonkarzinomzellen wurde beobachtet, dass deren Fähigkeit, eine extrazelluläre Matrix zu durchwandern, vom Sättigungsgrad der uPA-Rezeptoren mit aktivem uPA abhängig ist (Hollas et al., Cancer Res. 51 (1991), 3690-3695). Ebenfalls im Zellkulturmodell wurde eine Reduktion des invasiven Potentials von Zellen beobachtet, wenn die proteolytische Aktivität von uPA durch PAI-1 (Cajot et al., Proc. Natl. Acad. Sci. USA 87 (1990), 6939-6943) oder PAI-2 (Baker et al., Cancer Res. 50 (1990), 4676-4684) gehemmt wurde. Ein vergleichbarer Effekt wurde bei Hemmung der Bindung von uPA an die Zelloberfläche durch Blockierung des Rezeptors mittels proteolytisch inaktiver uPA-Varianten erzielt (Cohen et al., Blood 78 (1991), 479-487; Kobayashi et al., Br. J. Cancer 67 (1993), 537-544). Auch die Transfektion epidermoider Karzinomzellen mit einem Plasmid, das ein Antisense-Transkript gegen einen Teil von uPAR exprimiert, führte durch Unterdrückung der uPAR-Synthese zur Verringerung der Invasivität dieser Zellen (Kook, EMBO J. 13 (1994), 3983-3991). Gegen uPA und PAI-1 gerichtete Antikörper reduzierten das invasive Potential von Lungenkrebszellen in vitro (Liu et al., Int. J. Cancer 60 (1995), 501-506).

Der Einfluss des Plasminogenaktivator-Systems auf den Metastasierungsprozess konnte auch in Tumor-Tiermodellen belegt werden. So wurden die durch menschliche Karzinomzellen verursachte Bildung von Lungenmetastasen in Hühnerembryos durch Zugabe von Antikörpern gegen uPA fast vollständig verhindert (Ossowski und Reich, Cell 35 (1983), 611-619). Metastasierende menschliche Karzinomzellen wurden mit einem Expressionsplasmid transfiziert, das für eine proteolytisch inaktive, aber uPAR-bindende uPA-Mutante kodierte. Im Mausmodell zeigte sich, dass die Karzinomzellen, die inaktives uPA synthetisierten, nach Injektion im Vergleich zu den nichttransfizierten Zellen eine signifikant geringere Anzahl an Metastasen bildeten (Crowley et al., Proc. Natl. Acad. Sci. USA 90 (1993), 5021-5025). Nach Verabreichung von uPA-Antisense Oligonukleotiden wurde darüber hinaus eine Inhibierung der intraperitonealen Ausbreitung von humanen Ovarialkarzinomzellen in Nacktmäusen beobachtet (Wilhelm et al., Clin. Exp. Metast. 13 (1995), 296-302).

In den letzten Jahren wurde die klinische Relevanz von Faktoren des Plasminogenaktivator-Systems (uPA, uPAR, PAI-1 und PAI-2) für die Prognose von Patienten mit soliden malignen Tumoren intensiv untersucht. Dabei erwies sich der uPA-Antigengehalt bei verschiedenen Tumoren (z. B. Brust, Eierstock, Magen, Lunge, Niere etc.) sowohl für das rezidivfreie Überleben als auch für das Versterben als ein starker Prognosefaktor (siehe beispielsweise Schmitt et al., J. Obstet. Gynaecol. 21 (1995), 151-165; Jaenicke et al., Breast Cancer Res. Treat. 24 (1993), 195-208; Kuhn et al., Gynecol. Oncol. 55 (1994), 401-409; Nekarda et al., Lancet 343 (1994), 117; Pe-dersen et al., Cancer Res. 54 (1994), 4671-4675). Ebenso korrelieren erhöhte Konzentrationen an uPAR in Lungen- (Pedersen et al., supra) und Brustkrebsgewebe (Duggan et al., Int. J. Cancer 61 (1995), 597-600; Ronne et al., Breast Cancer Res. Treat. 33 (1995), 199-207) sowie bei Magenkrebs sowohl im Tumorgewebe selbst (Heiss et al., J. Clin. Oncol. 13 (1995), 2084-2093) als auch bei den ins Knochenmark ausgestreuten Tumorzellen (Heiss et al., Nature Medicine 1 (1995), 1035-1039) mit einer schlechten Prognose.

Der Einsatz von sythetischen uPA-Inhibitoren bietet die Möglichkeit, die Invasion und Ausbreitung von Tumorzellen zu unterdrücken. Allerdings ist die Entwicklung spezifischer uPA-Inhibitoren mit Schwierigkeiten behaftet, da der Plasminogenaktivator von Gewebetyp (tPA) eine identische Spezifität für die Spaltung der Peptidbindung Arg560/Val561 von Plasminogen aufweist. In den meisten Fällen hemmen daher niedermolekulare uPA-Inhibitoren auch tPA und damit auch tPA-vermittelte Fibrinolyse. Außerdem muss gewährleistet sein, dass synthetische uPA Inhibitoren keine starke Hemmung von Plasmin zeigen.

Trotz dieser Einschränkungen sind einige Hemmstoffe bekannt, die eine gewisse Spezifität gegenüber uPA, jedoch geringe inhibitorische Kapazität besitzen, wie etwa Benzamidin- und β-Naphthamidin-Derivate, wobei die wirksamste Verbindung uPA mit Kᵢ=2,2 µmol/l hemmt (Stürzebecher und Markwardt, Pharmazie 33 (1978), 599), oder Amilorid mit einem Kᵢ=7 µmol/l (Vassalli und Belin, FEBS. Lett. 214 (1987), 187-191).

DE-A-30 35 086 offenbart Cyclohexancarbonsäurederivate, die inhibitorische Wirkungen auf Proteasen wie Trypsin, Chymotrypsin, Thrombin oder uPA haben. Die untersuchten Verbindungen zeigen jedoch nur eine recht geringe und darüber hinaus unspezifische uPA-Inhibierung. EP-A-0 183 271 offenbart Lysinderivate und deren Verwendung als Proteaseinhibitoren. Es wird auch ein Benzamidino-Lysinderivat (Verbindung 108) beschrieben, das in vitro eine uPA-Hemmung, jedoch auch eine vergleichbare Wirkung auf andere Proteasen wie Trypsin oder Plasma-Kallikrein aufweist. WO 95/17885 offenbart niedermolekulare Polypeptide als uPA-Inhibitoren.

Eine weitere Klasse von bekannten uPA-Inhibitoren stellen 4-substituierte Benzothiophen-2-carboxamidine mit einem Kᵢ=0,16 mmol/l im Falle von Benzothiophen-623 dar (Towle et al., Cancer Res. 53 (1993), 2553-2559). Diese Hemmstoffe haben eine signifikant höhere Affinität für uPA im Vergleich zu tPA und Plasmin. Auch uPAR-gebundenes uPA wird mit hoher Effizienz gehemmt. Ein Nachteil dieser Substanzen liegt allerdings darin, dass die chemische Synthese kompliziert ist und kaum Möglichkeiten für die Modifizierung der Struktur vorhanden sind, bzw. bisher gezeigt werden konnte.

Die Entwicklung weiterer Hemmstoffe von uPA ist daher für die weitere Aufklärung der Rolle von uPA und uPAR bei verschiedenen Krankheiten, speziell bei der Tumorausbreitung und Metastasierung, von großem Nutzen.

Nα-Arylsulfonyl- und Nα-Arylsulfonyl-amino-acyl-Derivate des 3-Amidinophenylalanins sind als selektive Hemmstoffe von Thrombin (Markwardt et al., Thromb. Res. 17 (1980), 425-431) bzw. von Gerinnungsfaktor Xa (Stürze-becher et al., Thromb. Res. 54 (1989), 245-252) bekannt. Auch in WO 92/08709, WO 94/18185 und WO 96/05189 wird die Verwendung von Amidinophenylalaninderivaten als Hemmstoffe für die Blutgerinnung, insbesondere als Hemmstoffe für Thrombin, offenbart.

Intensiv wurden Piperidide und Piperazide des 3-Amidinophenylalanins untersucht, unter denen auch Leitstrukturen zur Hemmung fibrinolytischer Enzyme gefunden wurden (Stürzebecher et al., J. Enzyme Inhibition 9,87-99, 1995; Stürzebecher et al., J. Med. Chem. 40, 3091-3099, 1997). Während bei Stürzebecher et al. (1995) nur eine Hemmung von Thrombin, Faktor Xa, Plasmin und Trypsin beschrieben ist, finden sich bei Stürzebecher et al. (1997) auch Angaben zur Hemmung von uPA. Bei Nα-2-Naphthylsulfonyl-, Nα-2-(2,2,5,7,8-Pentamethylchroman-6-yl)sulfonylund Nα-2-Campher-10-yl-sulfonyl-substituierten 3-Amidinophenylalaninpiperaziden wird für uPA ein Kᵢ-Wert von 28 bis 140 µmol/l gefunden, der um drei Größenordnungen höher als die Inhibitionskonstante für Thrombin ist. Somit konnte nicht davon ausgegangen werden, dass 3-Amidinophenylalaninderivate als Urokinaseinhibitoren geeignet sind.

Überraschenderweise wurde jedoch gefunden, dass an Position 2 mit einem Phenylrest substituierte 3-Amidinophenylalaninderivate selektive und *in vivo* wirksame Hemmstoffe von uPA darstellen. Weiterhin wurde gefunden, dass diese Substanzen eine hohe Selektivität für Lymphgewebe aufweisen und sich daher als Mittel zum Targeting von Lymphzellen, beispielsweise zur Behandlung von malignen Erkrankungen des Lymphgewebes, wie etwa Lymphomen, eignen.

Die vorliegende Erfindung betrifft den neuen Urokinase-Inhibitor Nα-(2,4,6-Triisopropyl-phenylsulfonyl)-3-amidino-(D,L)-phenylalanin-4-ethoxycarbonyl-piperazid der allgemeinen Formel I, 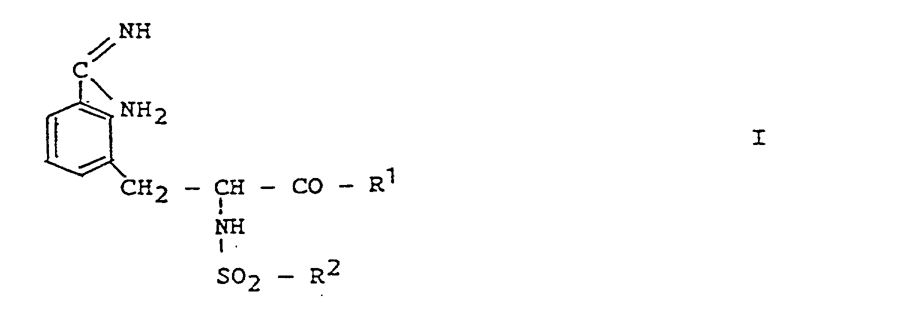 der als Racemate sowie als L- bzw. D-konfigurierte Verbindung vorliegt und in der
(a) eine Gruppe der Formel 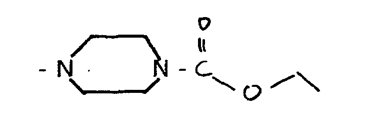 darstellt, und
   - R²: 2,4,6-Triisopropyl-phenyl, darstellt.

Die Verbindung kann auch als Salz vorzugsweise als physiologisch verträgliches Säuresalz, z.B. als Salz von Mineralsäuren, besonders bevorzugt als Hydrochlorid, oder als Salz von geeigneten organischen Säuren vorliegen.

Die Verbindung der Formel I kann in prinzipiell bekannter Weise, wie z.B. in WO 92/08709 und WO 94/18185 beschrieben, hergestellt und auf ihre biologische *in vitro* Aktivität gesetzt werden.

(L)-, (D)- oder (D,L)-3-Cyanphenylalanin-methylester-hydrochlorid wird mit einem entsprechenden Sulfochlorid oder einer sulfonylierten Aminosäure bzw. deren Halogenid in Gegenwart einer Base zu einer Verbindung der allgemeinen Formel I mit Cyanfunktion, in der R¹ = OCH₃ ist und R² darin der Beschreibung definierten Bedeutung entspricht, umgesetzt. Durch milde saure oder alkalische Hydrolyse sind daraus die Verbindungen der allgemeinen Formel I mit Carbonsäurestruktur (R¹ = -OH) erhältlich, deren Veresterung mit einem entsprechenden Alkohol unter säurekatalytischen Bedingungen zu Verbindungen der allgemeinen Formel I führt, wobei R¹ = (a) bedeutet. Nach einem in der Peptidchemie üblichen Verfahren, z.B. DCC-Verfahren in Gegenwart von HOBt, sind durch Umsetzung der Carbonsäuren der allgemeinen Formel I (R¹ = -OH) mit einem Nukleophil der Struktur (a) die Verbindungen mit entsprechendem R' der allgemeinen Formel I vorstellbar.

Die Zielverbindung mit der Formel I mit Amidinstruktur ist aus den Cyanverbindungen in bekannter Weise erhältlich, wobei in der Regel durch Addition von H₂S an die Cyangruppe zunächst die Thioamide erhalten werden, die durch S-Methylierung mit Methyliodid in die Thioimidoester und anschließend durch Behandlung mit Ammoniumacetat in alkoholischer Lösung in die Amidinoverbindungen überführt werden. Außerdem können gegebenenfalls aus den Cyanverbindungen mit Methanol oder Ethanol in Gegenwart von HCl-Gas und in bestimmten Fällen eines inerten Lösungsmittels die entsprechenden Imidoesterhydrochloride dargestellt werden, deren Umsetzung in alkoholischer Ammoniaklösung zu den Amidinoverbindungen führt.

Der erfindungsgemäße Urokinaseinhibitor kann gegebenenfalls zusammen mit mindestens einem geeigneten pharmazeutischen Hilfs- oder Trägerstoff zur Herstellung von oral, subkutan oder intravenös verabreichbaren Arzneimitteln zur Tumorbekämpfung oder in der Diagnostik verwendet werden. Ebenfalls möglich ist die Verabreichung in Kombination mit anderen Wirkstoffen, z.B. anderen Urokinaseinhibitoren, wie etwa Antikörpern oder/und Peptiden.

Die Arzneimittel zur Tumorbekämpfung bei Menschen und Tieren können topisch, oral, rektal oder parenteral, z.B. subkutan oder intravenös, in Form von Tabletten, Dragees, Kapseln, Pellets, Suppositorien, Lösungen oder transdermalen Systemen, wie Pflastern, verabreicht werden.

Nα-(2,4,6-Triisopropyl-phenylsulfonyl)-3-amidino-(D,L)-phenylalanin-4-ethoxycarbonyl-piperazid-hydrochlorid bzw. das L-Entantiomere davon oder ein pharmazeutisch verträgliches Salz dieser Verbindungen haben ein gutes Löslichkeitsverhalten. In Trispuffer (pH 7,3) sind sie bis zu einer Konzentration von 5 x 10⁻⁵ mol/l löslich. Durch Zusatz von 5 % Ethanol steigt die Löslichkeit auf 2x10⁻⁴mol/l und bei Zusatz von 5 % DMSO auf 10⁻³ mol/l.

Die erfindungsgemäße Verbindung ist in der Lage, hocheffizient das Wachstum oder/und die Ausbreitung von malignen Tumoren zu hemmen, z.B. die Tumorausbreitung beim Pankreaskarzinom, das Tumorwachstum des Mammakarzinoms sowie die Metastasierung von Tumoren. Dabei können die uPA-Inhibitoren gegebenenfalls zusammen mit anderen Antitumormitteln oder mit anderen Behandlungsarten, z.B. Bestrahlung oder chirurgischen Eingriffen, eingesetzt werden. Weiterhin sind die erfindungsgemäßen Inhibitoren auch für andere uPA-assoziierte Erkrankungen wirksam (z.B. bei der Verhinderung der Blasenbildung bei der Hauterkrankung Pemphigus vulgaris).

Erfindungsgemäße uPA Inhibitoren sind vorzugsweise dadurch gekennzeichnet, dass sie einen mindestens zweifach, vorzugsweise mindestens 5fach und besonders bevorzugt mindestens 10fach geringeren Kᵢ-Wert für uPA gegenüber tPA aufweisen. Weiterhin ist bemerkenswert, dass die erfindungsgemäßen Verbindungen die Blutgerinnung nur geringfügig beeinflussen, da sie für eine effektive Hemmung von Thrombin und Faktor Xa zu hohe Kᵢ-Werte haben.

Die erfindungsgemäße Substanz der Formel I kann in Form von Konjugaten mit physiologisch wirksamen Substanzen eingesetzt werden, z.B. mit Radiomarkierungen oder mit zytotoxischen Mitteln, z.B. Chemotherapeutika, wie cis-Platin oder 5-Fluor-Uracil, oder Peptiden.

Weiterhin kann die Substanz auch in die Membran von Trägervesikeln, z.B. Liposomen, eingebaut werden und somit ein Targeting von in den Trägervesikeln eingeschlossenen Wirksubstanzen, z.B. zytotoxischen Mitteln wie etwa Doxorubicin ermöglichen.

Eine weitere Indikation für die erfindungsgemäßen Verbindungen ist das Targeting von Lymphzellen, welches aufgrund ihrer um den Faktor 10 bis 20 höheren Affinität für Lymphknotengewebe gegenüber anderen Gewebetypen möglich wird. Somit eignen sich diese Substanzen hervorragend als Mittel zur Diagnostik oder zur Behandlung von Erkrankungen des Lymphgewebes, insbesondere malignen Erkrankungen, wie etwa Tumormetastasen und Lymphomen. Die Verabreichung der Substanzen kann wie zuvor bereits beschrieben erfolgen. Eine Behandlung von Erkrankungen des Lymphgewebes erfolgt vorzugsweise durch eine mehrtägige Verabreichung des Medikaments, z.B. über einen Zeitraum von 5 bis 20 Tagen, mit einer anschließenden Behandlungspause und gegebenenfalls ein oder mehrmaligem Wiederholen der Applikation.

Die Erfindung soll an den folgenden Beispielen und Abbildungen näher erläutert werden. Es zeigen:
- **Figur 1**: das Ergebnis einer Zytotoxizitätsbestimmung einer erfindungsgemäßen Substanz,
- **Figur 2**: das Ergebnis eines Versuchs zur Inhibierung des Abbaus einer Fibrinmatrix durch humane Mammakarzinomzellen,
- **Figuren 3 und 4**: die Wirkung einer erfindungsgemäßen Substanz auf die Ausbreitung, das Wachstum und die Metastasierung von Mammakarzinomzellen in der Ratte,
- **Figur 5**: die Wirkung einer erfindungsgemäßen Substanz auf das Wachstums eine Pankreastumors in der Ratte und
- **Figur 6**: die Wirkung einer erfindungsgemäßen Substanz auf das Wachstum humaner Mammakarzinomzellen in Mäusen.

### Beispiele

### 1. Nα-2,4,6-Triisopropylphenylsulfonyl-(L)-3-amidino-phenylalanin-4-ethoxycarbonyl-piperazid-hydrochlorid (erfindungsgemäße Verbindung:

### 1.1 Nα-2,4,6-Triisopropylphenylsulfonyl-(L)-3-cyan-phenylalaninmethylester

5 g (L)-3-Cyanphenylalanin-methylester wurden in 100 ml Dioxan suspendiert, 4,45 ml N-Methylmorpholin (NMM) zugefügt und 30 min gerührt. Nach Zugabe von 5,97 g 2,4,6-Triisopropylbenzolsulfochlorid in fester Form wurde 3 Tage gerührt, danach ausgefallenes NMM-HCI abfiltriert, das Lösungsmittel abdestilliert und das erhaltene Rohprodukt über Kieselgel (KG) 60 (Chloroform) gereinigt. Ausbeute: 8,34 g Sirup (90 %).

### 1.2 Nα-2,4,6-Triisopropylphenylsulfonyl-(L)-3-cyan-phenylalanin

8,34 g der Verbindung 1. 1 wurden in einer Mischung aus je 50 ml Essigsäure und 1 N Salzsäure 8 Std. unter Rückfluß erhitzt, nach dem Erkalten 2x mit Ethylacetat extrahiert, die vereinigten Ethylacetatlösungen über MgSO₄ getrocknet und das Lösungsmittel abdestilliert. Nach Reinigung über KG 60 (Chloroform) wurden 5,8 g eines festen Produktes erhalten (72 %).

### 1.3 Nα-2,4,6-Triisopropylphenylsulfonyl-(L)-3-cyan-phenylalanin-4-ethoxycarbonyl-piperazid

5,7 g der Verbindung 1.2 wurden in 100 ml Tetrahydrofuran (THF) gelöst, auf 0 °C abgekühlt, 2,22 g α-Hydroxybenzotriazol (HOBt), 2,82 g Dicyclohexylcarbodiimid (DCC) zugefügt und 30 min gerührt. Nach Zugabe von 3,94 g 1-Ethoxycarbonyl-piperazin in 30 ml THF wurde über Nacht gerührt, danach ausgefallenes Dicyclohexylurea (DCU) abfiltriert, das Lösungsmittel abdestilliert und das erhaltene Rohprodukt über KG 60 (Chloroform) gereinigt. Ausbeute: 7,1 g eines amorphen Pulvers (96 %).

### 1.4 Nα-2,4,6-Triisopropylphenylsulfonyl-(L)-3-amidino-phenylalanin-4-ethoxycarbonyl-piperazid Hydrochlorid

7,1 g der Verbindung 1.3 wurden in 30 ml Pyridin gelöst, 30 Tropfen Triethanolamin (TEA) zugefügt, 10 min ein kräftiger Schwefelwasserstoffstrom eingeleitet und 2 Tage bei Raumtemperatur stehengelassen. Anschließend wurde das Lösungsmittel abdestilliert, der Rückstand in Ethylacetat gelöst, die organische Phase mit 1 N Salzsäure und gesättigter Kochsalzlösung gewaschen, über MgSO₄ getrocknet und das Lösungsmittel abdestilliert. 7,2 g des auf diese Weise erhaltenen Thioamids wurden in 250 ml Aceton gelöst, die Lösung mit 17 g Methyliodid versetzt und 2 Tage bei Raumtemperatur unter Lichtschutz stehengelassen. Danach wurde das Lösungsmittel abdestilliert, das Thioimidesterhydroiodid (8,5 g) in 50 ml Methanol gelöst, 1,9 g Ammoniumacetat zugefügt und der Ansatz 4 Std. bei 60 °C erwärmt. Das nach Abdestillieren des Lösungsmittels erhaltene Rohprodukt wurde über Sephadex LH20 (Methanol) gereinigt. Das auf diese Weise erhaltene Amidin-hydroiodid wurde über Ionenaustauscher (Amberlite IRA-420) in das Hydrochlorid übergeführt. Ausbeute: 5,3 g eines amorphen Pulvers (69 %).

### 2. Nα-2,4,6-Triisopropylphenylsufonyl-(D,L)-3-amidinopenyl-alanylnipecotinsäure-benzylamid Hydrochlorid (vergleichsbeispriel)

### 2.1 Nα-2,4,6-Triisopropylphenylsulfonyl-(D,L)-3-cyan-phenylalanylnipecotinsäure-ethylester

4,56 g Nα-2,4,6-Triisopropylphenylsulfonyl-(D,L)-3-cyanphenylalanin(aus(D,L)-3-Cyanphenylalanin-methylester-hydrochlorid und dem entsprechenden Sulfochlorid analog 1.1 und 1.2 dargestellt), 1,5 g HOBt und 2,42 g DCC wurden in 50 ml DMF gelöst, 1 Std. gerührt und danach 2,36 g Nipecotinsäure-ethylester zugefügt. Nach Rühren über Nacht wurde ausgefallenes DCU abfiltriert, das Lösungsmittel abdestilliert, der Rückstand in wenig Methanol gelöst und zur Kristallisation stehengelassen. Der gebildete Niederschlag wurde abgesaugt, mit Methanol gewaschen und getrocknet. Ausbeute: 4,46 g (75 %).

### 2.2 Nα-2,4,6-Triisopropylphenylsufonyl-(D,L)-3-cyan-phenylalanylnipecotinsäure

4,4 g des vorher beschriebenen Ethylesters wurden in einer Mischung aus 35 ml Essigsäure und 25 ml 1 N HCI 2 Std. unter Rückfluß erhitzt. Nach Zugabe von 10 ml Wasser wurde zum Erkalten stehengelassen, wobei sich ein wachsartiges Produkt abschied. Nach Abgießen des Lösungsmittels wurden 200 ml Wasser zugesetzt, längere Zeit kräftig gerührt und die erhaltene feste Substanz abgesaugt, mit Wasser gewaschen und getrocknet. Ausbeute: 3,84 g (92 %).

### 2.3 Nα-2,4,6-Triisopropylphenylsulfonyl-(D,L)-3-cyan-phenylalanylnipecotinsäure-benzylamid

2,28 g der vorher beschriebenen Verbindung 0,6 g HOBt und 0,97 g DCC wurden in 20 ml DMF gelöst, 1 Std. gerührt, anschließend 0,6 g Benzylamin zugefügt und weiter über Nacht gerührt. Nach Abfiltrieren des ausgefallenen DCU wurde das Lösungsmittel abdestilliert, der Rückstand in Methanol gelöst und die Lösung in 5-proz. Natriumhydrogencarbonatlösung/Eis gegossen. Nach 1 Std. wurde der gebildete Niederschlag abgesaugt, mit Wasser gewaschen und im Vakuum getrocknet. Ausbeute: 2,48 g (94 %).

### 2.4 Nα-2,4,6-Triisopropylphenylsulfonyl-(D,L)-3-amidino-phenylalanylnipecotinsäure-benzylamid Hydrochlorid

2,4 g der Verbindung 2.3 wurden in 30 ml Pyridin gelöst, 30 Tropfen TEA zugefügt, in die Lösung 10 min Schwefelwasserstoff eingeleitet und der Ansatz 2 Tage bei Raumtemperatur stehengelassen. Anschließend wurde das Lösungsmittel abdestilliert, der Rückstand in Ethylacetat aufgenommen und mit 1 N HCl ausgeschüttelt. Nach Waschen der organischen Phase mit gesättigter Kochsalzlösung und Trocknen über Natriumsulfat wurde das Lösungsmittel abdestilliert. 2,38 g des auf diese Weise erhaltenen Thioamids wurden in 100 ml Aceton gelöst, die Lösung mit 6,5 g Methyliodid versetzt und 20 Std. bei Raumtemperatur unter Lichtschutz stehengelassen. Danach wurde das Lösungsmittel abdestilliert, das Thioimidonester-hydroiodid in 50 ml Methanol gelöst, 0, 5 g Ammoniumacetat zugegeben und der Ansatz 4 Std. bei 60 °C im Wasserbad erwärmt. Das nach Abdestillieren des Lösungsmittels erhaltene Rohprodukt konnte über KG 60 gereinigt werden. Die Elution erfolgte zunächst mit Chloroform, danach mit Chloroform/Methanol 9:1. Das so gereinigte Amidin-hydroiodid wurde über lonenaustauscher (Amberlite IRA-420) in das Hydrochlorid übergeführt. Ausbeute: 1,45 g eines amorphen Pulvers (56 %).

Die Charakterisierung der Verbindungen erfolgte massenspektrometrisch, eine Reinheitsprüfung erfolgte mittels DC und HPLC.

### 3. In vitro Hemmung von Urokinase durch ausgewählte Verbindungen der Formel I

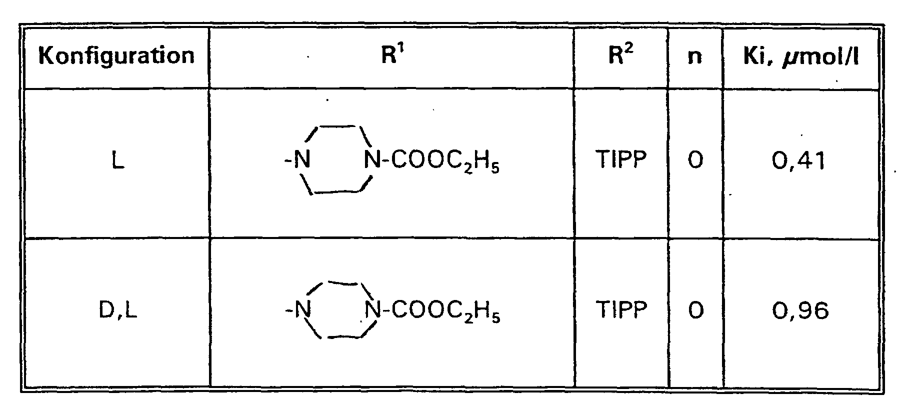

Abkürzungen: TIPP - 2,4,6-Triisopropylphenyl

### Bestimmung der Hemmwirkung

Zur Bestimmung der Inhibitoraktivität wurden 200 µl Tris-Puffer (0,05 mol/l, den Inhibitor enthaltend, 0,154 mol/l NaCI, 5 % Ethanol pH 8,0), 25 µl Substrat (Pefachrome UK oder Bz-βAla-Gly-Arg-pNA in H₂O; Pentapharm Ltd., Basel, Schweiz) und 50 µl sc-Urokinase (Ribosepharm GmbH, Haan, Deutschland) bei 25 °C inkubiert. Nach 3 min wurde die Reaktion durch Zugabe von 25 µl Essigsäure (50 %) unterbrochen und die Absorption bei 405 nm mittels Microplate Reader (MR 5000, Dynatech, Denkendorf, Deutschland) bestimmt. Die Kᵢ-Werte wurden nach Dixon durch lineare Regression mittels eines Computerprogramms ermittelt. Die Kᵢ-Werte sind das Mittel aus mindestens 3 Bestimmungen, die Standardabweichung lag unter 25 %.

### 4. In vitro Hemmung verschiedener Serinproteasen vom Trypsin-Typ durch (Nα-(2,4,6-Triisopropyl-phenylsulfonyl)-(L)-3-amidino-phenylalanin-4-ethoxycarbonyl-piperazid(uPA-Inhibitor)undNα-2-Naphthylsulfonyl-3-amidinophenylalanin-N'-methylpiperazid (Napththylsulfonyl-Derivat) als Vergleich

| | Ki [µmol/l] | |
|---|---|---|
| **Enzym** | **uPA-Inh.** | **Naphthylsulfonyl-Der.** |
| Urokinase | 0,41 | 150 |
| Plasmin | 0,39 | 55 |
| Sc-tPA | 4,9 | 430 |
| Thrombin | 0,49 | 0,036 |
| Faktor Xa | 1,7 | 30 |
| Faktor XIIa | 13 | > 1000 |
| Plasma-Kallikrein | 7,2 | 85 |
| Glandulär-Kallikrein | > 1000 | > 1000 |
| Trypsin | 0,037 | 1,3 |
| Tryptase | 6,3 | 33 |

Die Bestimmung der Hemmwirkung für die verwendeten Enzyme erfolgte nach dem in Beispiel 3 beschriebenen Prinzip.

Aus den oben angegebenen Werten ist ersichtlich, dass der erfindungsgemäßeUrokinasehemmstoffuPA-Inhibitorüberraschenderweise einen um mehr als den Faktor 10 kleineren Kᵢ-Wert für Urokinase als für Einzelketten tPA (Sc-tPA) aufweist. Somit eignen sich die erfindungsgemäßen Substanzen als selektive Urokinaseinhibitoren. Zum Vergleich ist die Inhibitoraktivität des Naphthylsulfonyl-Derivats angeführt, das eine signifikant geringere *in vitro* Anti-uPA-Aktivität aufweist.

### 5. Bestimmung der Zytotoxizität

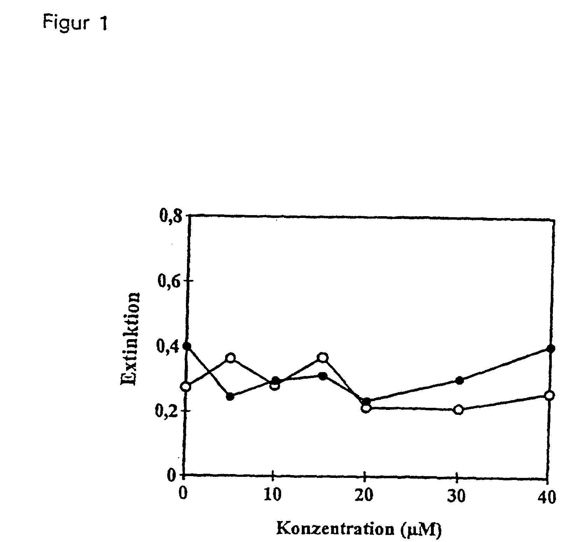

Für die Bestimmung der Zellproliferation/Zytotoxizität wurde ein kommerziell erhältlicher Test eingesetzt (Promega), welcher auf der zellulären Umsetzung eines Tetrazoliumsalzes beruht. Das aus dieser Reaktion resultierende farbige Produkt kann mittels eines ELISA-Spektrometers (ICN-Flow) quantifiziert werden. Der synthetische Inhibitor (offene Kreise) hatte gegenüber dem Lösungsmittel (geschlossene Kreise) alleine keinen Einfluss auf das Zellwachstum humaner Orvarialkarzinomzellen OV-MZ-6 (Figur 1). Somit ist der erfindungsgemäße uPA-Inhibitor in pharmakologisch wirksamen Konzentrationen bis 40 µM nicht zytotoxisch.

### 6. Inhibierung des Abbaus einer Fibrin-Matrix durch humane Mammakarzinomzellen

Zur Untersuchung der Fähigkeit von Tumorzellen eine extrazelluläre Matrix abzubauen, wurde ein Fibrinmatrixabbau-Test entwickelt und verwendet. Je größer die proteolytische Aktivität der Tumorzellen, um so höher ist die Konzentration der Fibrinabbauprodukte im Matrixüberstand. Die Matrixabbaukapazität entspricht der Konzentration der Fibrinabbauprodukte, welche mittels ELISA (D-Dimer) ermittelt werden.

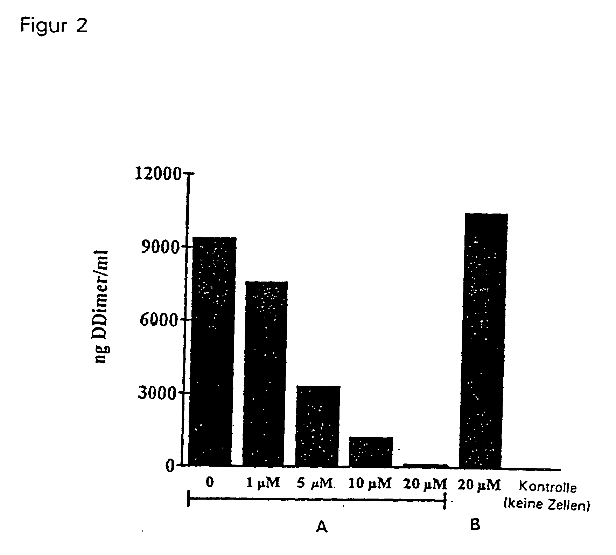

Die Fibringele wurden in 24 Well Kulturplatten aus 200 ml Fibrinogen (50 mg/ml) in PBS (pH 7,4) durch 50 µl Thrombin (10 U/ml) und 50 µl CaCl₂ (150 mM) pro Well nach Inkubation für 30 min bei 37 °C gebildet. 2 x 10⁵ Mammakrzinomzellen wurden auf diese Fibrinmatrix in 1 ml DMEM Kulturmedium, plus 10 % fetalem Kälberserum und 2 µg Glu-Plasminogen ausgesät und 4 Std. lang inkubiert. Danach wurde der Überstand zentrifugiert, um Zellen zu entfernen, und die Fibrinabbauprodukte mittels ELISA quantifiziert. Die Zugabe des erfindungsgemäßen Inhibitors (A) in unterschiedlichen Konzentrationen führte zu einer signifikanten Inhibierung des Matrixabbaus durch Mammakarzinomzellen im Vergleich zu dem Naphthyl-Derivat (B), das keine Hemmung des Fibrinabbaus durch Mammakarzinomzellen zeigt (Figur 2).

### 7. In vivo Test des uPA Inhibitors auf Tumorausbreitung, Tumorwachstum und Metastasierung in der Ratte

### A) Brustkrebs-Modell

10-25 mm³ Rattenbrustkrebs-Tumorfragmente BN-472 wurden subkutan orthotropisch unter die Fettpolster der Brustdrüse in 6 bis 7 Wochen alten, weiblichen Brown Norwegian Ratten transplantiert (Tag 0). Die Behandlung der Tiere wurde 24 h nach der Tumorinokulation intraperitoneal begonnen. Jede Gruppe bestand aus 8 Tieren. Die Kontrollgruppe erhielt nur Injektionslösung (100 µl einer 10 % Ethanol/Saline (0,9 % NaCl) Lösung). Der Vergleichsgruppe des Naphthyl-Derivats (B) und der Therapiegruppe des erfindungsgemäßen uPA-Inhibitors (A) wurden in der oben genannten Lösung eine Dosis von 1 mg/kg Körpergewicht täglich i.p. verabreicht. Die Behandlung wurde über einen Zeitraum von 4 Wochen durchgeführt.

Die Abmessungen der subkutanen Tumoren und die Gewichte der Tiere wurden wöchentlich bestimmt. Am Ende der Behandlung wurden die Tiere getötet und Tumorgewichte, Organgewichte und die Anzahl von Metastasen in relevanten Geweben bestimmt.

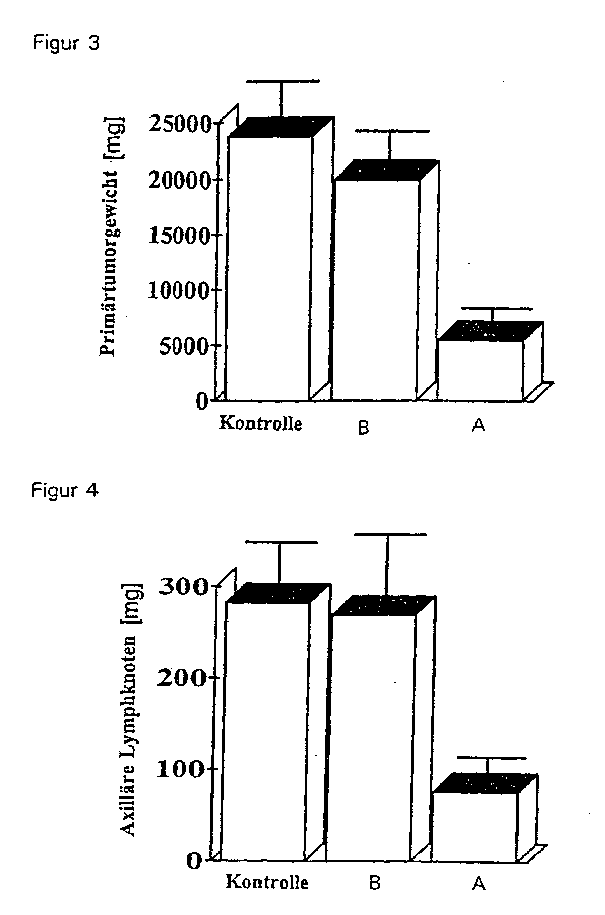

Die Behandlung mit uPA-Inhibitor (A) führte zu einer signifikanten Reduktion des Primärtumorgewichtes sowie der axillären Lymphknoten (p=0,003 bzw. p=0,005) im Vergleich mit dem Naphthyl-Derivat (B) und Kontrollgruppen ohne Inhibitor (Fig. 3 und 4). Die Gewichte von Lunge, Leber, Niere und Milz bei den mit dem uPA-Inhibitor behandelten Tieren waren unverändert gegenüber den Kontrolltieren.

### B) Pankreaskarzinom-Modell

Fragmente des transplantierbaren und metastasierenden Pankreasadenokarzinoms CA20948 der Ratte wurden aus Donortieren explantiert. Nach Zellvereinzelung wurden gleiche Mengen an suspendierten Tumorzellen zusammen mit 2 mg Matrigel jedem der Empfängertiere, männlichen 10 Wochen alten Lewisratten (n=9), subkutan implantiert. Die Durchführung der Behandlung sowie Zusammensetzung der Therapiegruppen war identisch wie unter A).

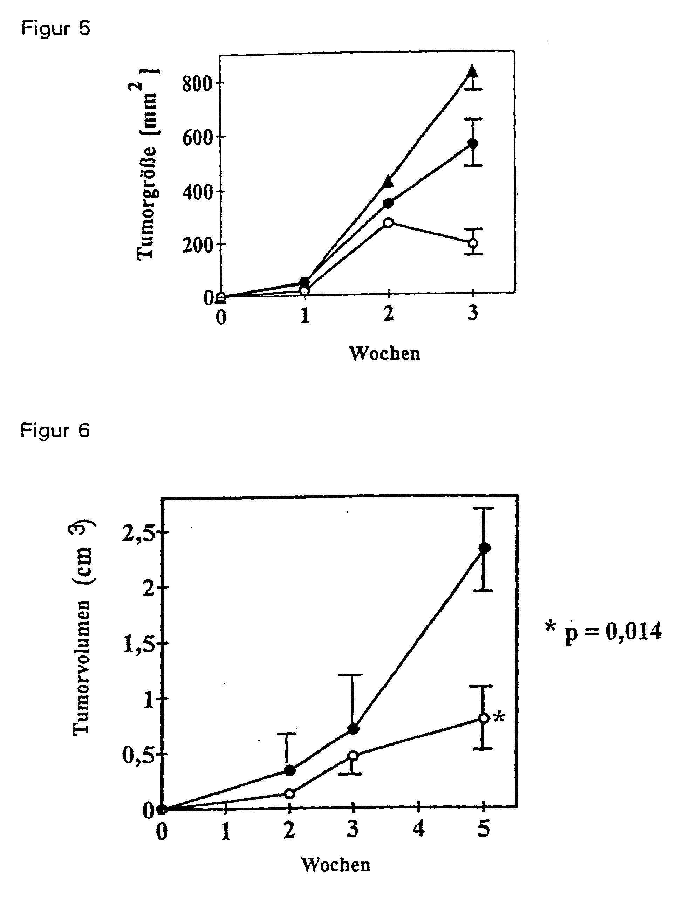

Wie aus Figur 5 ersichtlich ist, findet man beim erfindungsgemäßen uPA-Inhibitor (offene Kreise) gegenüber dem Naphthyl-Derivat (geschlossene Kreise) und der Kontrollgruppe (Dreiecke) eine signifikante Verringerung des Tumorgewichts und eine Verminderung des Wachstums bei den entstehenden Ratten-Pankreaskarzinom.

### C) Wiederholung der Versuche mit geänderter Applikationsart

Die in den Abschnitten A und B beschriebenen Versuche wurden mit geänderter Applikationsart des Inhibitors wiederholt. Dabei wurde ohne Veränderung der täglichen Dosis der Inhibitor im Mammakarzinom-Modell subkutan (n=9) und im Pankreasadeonkarzinom-Modell intraperitonial (n = 8) appliziert. Die Ergebnisse dieser Wiederholungsversuche entsprachen in Tendenz und Umfang den bereits dargestellten Ergebnissen.

### D) Zusammenfassung der Ergebnisse

In allen Versuchen wurde durch Behandlung mit dem Inhibitor eine erhebliche Reduktion der Tumorgröße bzw. des Tumorgewichts und der Anzahl bzw. der Masse von Tochtergeschwülsten im Vergleich zu den Kontrollgruppen erreicht. Im Mammatumor-Modell waren in der Inhibitor-behandelten Gruppe, die mittleren Tumorgewichte am Ende der Behandlung auf 23 % (i.p.) bzw. 37 % (auf s.c.) im Vergleich zu der mit Vehikel behandelten Kontrolle reduziert. Die Anzahl der Lungenfoci in Inhibitor-behandelten Gruppen waren auf 9 % (i.p.) bzw. 32 % (s.c.) und die mittleren Gewichte der axillären Lymphknoten auf 27 % (i.p.) bzw. 48 % (s.c.) reduziert.

Im Pankreastumor-Modell waren die Massen des tumorhaltigen Pankreas in den Inhibitor-behandelten Gruppen um 76 % (i.p.) bzw. 34 % (s.c.), die Massen der subkutanen Tumoren um 54 % (i.p.) bzw. 60 % (s.c.) verglichen mit den jeweiligen Vehikel-behandelten Gruppen reduziert. Die Anzahl detektierter Leberfoci in behandelten Gruppen war 29 % (i.p.), bzw. 2 % (s.c.) im Vergleich zu den Vehikel-behandelten Kontrollgruppen.

Die Entwicklung der Körpergewichtszunahme und der Vergleich der Organgewichte zwischen Inhibitor- und Vehikel-behandelten Gruppen ließen keine Hinweise für eine etwaige erhebliche Toxizität des Inhibitors unter den beschriebenen Bedingungen erkennen.

### 8. Behandlung humaner Brustkrebszellen in der Nacktmaus

Um die *in vivo* Effizienz des Inhibitors zur Hemmung des Tumorwachstums humaner Mammakarzinomzellen zu testen (MDA-BA-231), wurden 6 x 10⁶ Zellen subcutan in die rechte Flanke von Balb/c Nacktmäusen (4-6 Wochen alt) injiziert. Die Tumorzellen wurden vor Inokulation mit dem synthetischen uPA-Inhibitor vorinkubiert. Nach 24 h wurden die Mäuse mit einer Dosis von 1,2 mg/kg Körpergewicht intraperitoneal wie unter A) beschrieben zweimal wöchentlich behandelt. Die Tumorgröße wurde wöchentlich durch Messung der zwei größten Durchmesser bestimmt.

Wie aus Figur 6 ersichtlich ist, nimmt das Tumorvolumen bei der Verabreichung des uPA-Inhibitors (offene Kreise) signifikant langsamer zu als in der Kontrollgruppe (geschlossene Kreise) wo Ethanol in Saline verabreicht wurde.

### 9. Die Biodistribution des Inhibitors in der Ratte

Die Biodistribution des Inhibitors wurde in zwei unabhängigen Versuchen in Gewebeaufschlüssen von Ratten ermittelt, die 5 bzw. 10 Tage lang täglich einmal mit 1 mg/kg Inhibitor i.p. behandelt worden waren. Zum Aufschluss wurden jeweils 100 mg Gewebe mechanisch zerkleinert und mit 200 µl 1 % Triton X-100 in physiologischer Kochsalzlösung vermischt. Nach Zugabe von 400 µl Ethanol wurde der Aufschluß 1 min mit Ultraschall behandelt. Der Gewebeextrakt wurde 15 min bei 12.000 x g zentrifugiert. Zur Vorreinigung wurde der Überstand auf eine mit 1 ml Methanol und 1 ml Wasser equilibrierte C18-Silica Reversed Phase Vorsäule (Sep-Pak® cartridge C18, 1 ml Waters, Eschborn, Deutschland) aufgetragen, hintereinander mit 2 x 1 ml H₂O, 1 ml 10 % Methanol, 1 ml H₂O, 1 ml 5 % Acetonitril, 0,04 % Perchlorsäure und 1 ml H₂O gewaschen und mit 500 µl 75 % Acetonitril, 0,04 % Perchlorsäure eluiert. Die HPLC-Analytik wurde auf einer Reversed Phase C18 Silica Säule mit einem 5-55 %igen Acetonitril Gradienten mit 0,04 % Perchlorsäure durchgeführt.

Die Konzentrationen des Inhibitors in den jeweils untersuchten Gewebetypen (µg/g) sowie in Blutplasma und Galle (µg/ml) und zum Vergleich eines entsprechenden Naphthylsulfonylderivats sind in der nachfolgenden Tabelle dargestellt.

### Tabelle

Verteilungsprofil einer erfindungsgemäßen Substanz in verschiedenen Geweben bzw. Blutplasma und Galle im Vergleich zu einem Naphthylsulfonyl-Derivat (Nα-2-Naphthylsulfonyl-3-amidinophenylalanin-4-ethoxycarbonyl-piperazid)

| Gehalt (µg/g) | | | |
|---|---|---|---|
| Gewebe | uPA-Inhibitor 1mg/kg i.p. 5 Tage | uPA-Inhibitor 1 mg/kg i.p. 10 Tage | Naphthylsulfonyl-Drivat 1 mg/kg i.p., 5 Tage |
| Milz | 2,02 | 2,48 | 0,089 |
| Leber | 2,85 | 2,08 | 0,12 |
| Niere | 2,67 | 2,48 | 0,085 |
| Muskel | 0,74 | < 0,5 | 0,008 |
| Fett Niere | 0,82 | 1,0 | < 0.005 |
| Herz | <0,5 | 1,09 | 0,59 |
| Lunge | 3,70 | 1,81 | 0,020 |
| Gehirn | <0,5 | <0,5 | |
| Lymphknoten Trachea | 7,45 | 16,38 | 0,12 |
| Lymphknoten Achsel | 1,97 | 2,74 | < 0,005 |
| Lymphknoten Knie | 7,83 | 3,8 | < 0,005 |
| | Gehalt (µg/ml) | | |
| Plasma | 0,008 | 0,035 | 0,004 |
| Galle | 1,96 | 1,75 | 0,097 |

In den meisten untersuchten Gewebetypen lag nach 5 bis 10 Tagen der Inhibitor in einer Größenordnung von 1 bis 3 µg/g vor. Die Plasmakonzentrationen des Inhibitors lagen 24 h nach der jeweils letzten i.p. Applikation jeweils ein bis zwei Größenordnungen unter den mittleren Gewebekonzentrationen. Daraus kann auf eine hohe Affinität für Gewebe und eine niedrige Plasmaeiweißbindung geschlossen werden. Die Konzentrationen des im Vergleich über 5 Tage applizierten Naphthylsulfonyl-Derivats waren in den verschiedenen Geweben 20-30fach geringer.

Der Inhibitor zeigt eine auffallende Anreicherung in den Lymphknoten. In den unabhängigen Versuchen wurden in trachealen Lymphknoten nach 5tägiger Applikation Konzentrationen 5,3 bzw. 7,5 µg/g, nach 10tägiger Applikation 21,6 bzw. 16,4 µg/g gemessen. Da Tumorzellen sich regelmäßig über lymphatische Bahnen disseminieren, ist die spezifische Anreicherung des Inhibitors in den Lymphgefäßen von Bedeutung und Vorteil für dessen Einsatz als anti-metastatisches Therapeutikum.

## Patentansprüche

1. Arzneimittel,
**dadurch gekennzeichnet,**
**dass** es als Wirkstoff Nα-(2,4,6-Triisopropyl-phenylsulfonyl)-3-amidino-(D,L)-phenylalanin-4-ethoxycarbonyl-piperazid, das L-Enantiomerdavon oder ein pharmazeutisch verträgliches Salz einer der Verbindungen enthält.

2. Arzneimittel nach Anspruch 1 zur Verabreichung in Kombination mit anderen Wirkstoffen.

3. Arzneimittel nach Anspruch 1 oder 2 zur Verabreichung in Kombination mit anderen Antitumormitteln.

4. Arzneimittel nach Anspruch 2 oder 3 zur Verabreichung in Kombination mit anderen Urokinase-Inhibitoren.

5. Arzneimittel nach Anspruch 4,
**dadurch gekennzeichnet,**
**dass** die Urokinase-Inhibitoren ausgewählt sind aus der Gruppe bestehend aus Antikörpern oder/und Peptiden.

6. Arzneimittel nach Anspruch 1 zum Einsatz mit anderen Behandlungsarten, z.B. Bestrahlung oder chirurgischen Eingriffen.

7. Arzneimittel nach Anspruch 1,
**dadurch gekennzeichnet,**
**dass** es den Wirkstoff als Konjugat mit einer physiologisch wirksamen Substanz enthält.

8. Arzneimittel nach einem der Ansprüche 1 bis 7,
**dadurch gekennzeichnet,**
**dass** es des Weiteren mindestens einen pharmazeutisch geeigneten Hilfs- oder/und Trägerstoff enthält.

9. Arzneimittel nach einem der Ansprüche 1 bis 8 zur topischen, oralen, rektalen oder parenteralen, z.B. subkutanen oder intravenösen, Verabreichung.

10. Arzneimittel nach einem der Ansprüche 1 bis 9 in Form von Tabletten, Dragees, Kapseln, Pellets, Suppositorien, Lösungen odertransdermalen Systemen, wie z.B. Pflastern.

11. Arzneimittel nach Anspruch 1,
**dadurch gekennzeichnet,**
**dass** der Wirkstoff in die Membran von Trägervesikeln, z.B. Liposomen, eingebaut ist.

## Claims

1. Medicament, **characterized in that** it comprises as active compound Nα-(2,4,6-triisopropylphenylsulphonyl)-3-amidino-(D,L)-phenylalanine 4-ethoxycarbonylpiperazide, the L enantiomer thereof or a pharmaceutically acceptable salt of any of the compounds.

2. Medicament according to Claim 1 for administration in combination with other active compounds.

3. Medicament according to either of Claims 1 and 2 for administration in combination with other anti-tumour agents.

4. Medicament according to either of Claims 2 and 3 for administration in combination with other urokinase inhibitors.

5. Medicament according to Claim 4, **characterized in that** the urokinase inhibitors are selected from the group consisting of antibodies and/or peptides.

6. Medicament according to Claim 1 for use in other types of treatment, for example radiation or surgical interventions.

7. Medicament according to Claim 1, **characterized in that** it comprises the active compound as conjugate with a physiologically active substance.

8. Medicament according to any of Claims 1 to 7, **characterized in that** it furthermore comprises at least one pharmaceutically suitable excipient and/or carrier.

9. Medicament according to any of Claims 1 to 8 for topical, oral, rectal or parenteral, for example subcutaneous or intravenous, administration.

10. Medicament according to any of Claims 1 to 9 in the form of tablets, coated tablets, capsules, pellets, suppositories, solutions or transdermal systems such as plasters, for example.

11. Medicament according to Claim 1, **characterized in that** the active compound is incorporated in the membrane of carrier vesicles, for example liposomes.

## Revendications

1. Agent pharmaceutique **caractérisé en ce qu'**il contient comme agent actif, le N-α-(2,4,6-triisopropylphénylsulfonyl)-3-amidino-(D,L)-phénylalaline-4-éthoxycarbonylpipérazide, son énantiomère L ou un sel physiologiquement compatible d'un des composés.

2. Agent pharmaceutique selon la revendication 1, à administrer en combinaison avec d'autres agents actifs.

3. Agent pharmaceutique selon la revendication 1 ou 2, à administrer en combinaison avec d'autres agents antitumoraux.

4. Agent pharmaceutique selon la revendication 2 ou 3, à administrer en combinaison avec d'autres inhibiteurs de l'urokinase.

5. Agent pharmaceutique selon la revendication 4, **caractérisé en ce que** les inhibiteurs d'urokinase sont choisis parmi le groupe consistant en des anticorps et/ou des peptides.

6. Agent pharmaceutique selon la revendication 1, à mettre en oeuvre avec d'autres types de traitement, par exemple le rayonnement ou l'intervention chirurgicale.

7. Agent pharmaceutique selon la revendication 1, **caractérisé en ce qu'**il contient l'agent actif en tant que conjugué avec une substance physiologiquement active.

8. Agent pharmaceutique selon l'une quelconque des revendications 1 à 7, **caractérisé en ce qu'**il contient par ailleurs, au moins un auxiliaire et/ou support pharmaceutiquement appropriés.

9. Agent pharmaceutique selon l'une quelconque des revendications 1 à 8, pour une administration topique, orale, rectale ou parentérale, par exemple sous-cutanée ou intraveineuse.

10. Agent pharmaceutique selon l'une quelconque des revendications 1 à 9, sous la forme de comprimés, dragées capsules, pastilles, suppositoires, solutions ou systèmes transdermiques, comme par exemple des timbres (Pflastern).

11. Agent pharmaceutique selon la revendication 1, **caractérisé en ce que** l'agent actif est incorporé dans la membrane de vésicules support, par exemple des liposomes.
